# FASCICULE DE BREVET EUROPEEN

(11) **EP 4 099 902 B1**
(45) Date de publication et mention de la délivrance du brevet: **10.12.2025**
(21) Numéro de dépôt: 21704221.7
(22) Date de dépôt: 05.02.2021
(51) Int. Cl.: A61B 5/145, A61B 5/00

(54) **DISPOSITIF DE MESURE D'ANALYTE COMPRENANT UN PATCH ADHESIF**
GERÄT MIT HEFTPFLASTER ZUR MESSUNG EINES ANALYTS
ANALYTE MEASUREMENT DEVICE COMPRINSING AN ADHESIVE PATCH

(30) Priorité: 05.02.2020 EP 20305109
(43) Date de publication de la demande: 14.12.2022
(73) Titulaire: WIZP AS, 3125 Tønsberg (NO)
(72) Inventeur: PIERART, Luc, 94800 VILLEJUIF (FR)
(74) Mandataire: Regimbeau
(86) Numéro de dépôt international: PCT/EP2021/052834
(87) Numéro de publication internationale: WO 2021/156453

(56) Documents cités:
- WO-A1-2019/141743
- CN-U- 201 517 084
- CN-U- 205 108 090
- US-A1- 2012 010 642

## Description

### DOMAINE DE L'INVENTION

La présente invention se rapporte un système de surveillance corporelle via analyse de liquide corporel, typiquement interstitiel, à l'aide de microaiguilles.

Plus précisément, la présente invention concerne un patch pour la gestion du maintien des microaiguilles dans la peau.

### ETAT DE LA TECHNIQUE

Certaines pathologies comme le diabète nécessitent une surveillance quotidienne de paramètres biochimiques du corps humain, *i.e.* des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

On connait aussi le dispositif du document WO2018104647, qui présente un boitier comprenant une capsule amovible, la capsule accueillant des microaiguilles configurées pour prélever du liquide interstitiel. Le boitier, quant à lui, accueille la majeure partie de l'électronique. Un autre dispositif est connu du document WO2019141743.

Ces produits sont des patchs autonomes collés sur le haut du bras ou le ventreet nécessite un adhésif extrêmement fort pour résister à 7-14 jours de douches mais à l'avantage d'être sur une zone sans poils.

Ce dispositif portatif, typiquement au poignet, permet une mesure en continu et il suffit de changer la capsule pour changer de microaiguilles.

Toutefois, lorsqu'un tel dispositif est porté au poignet, la difficulté réside dans la conciliation d'un système permettant maintien des microaiguilles en position dans la peau et d'un système remplaçable, d'un système de tenue forte mais n'arrachant pas les poils du poignet.

En outre, il est important que le dispositif soit facilement utilisable. L'invention vise à adresser ces difficultés.

### EXPOSE DE L'INVENTION

Pour répondre à certaines de ces problématiques, l'invention propose un ensemble pour système de surveillance corporelle, comprenant :
- un patch pour système de surveillance corporelle, le patch comprenant un corps principal avec un premier orifice d'au moins 2 mm de diamètre,
   le corps principal comprenant une première face et un adhésif recouvrant la première face,
   le corps principal comprenant une deuxième face opposée à la première face, le premier orifice formant un ajour au travers de la première face et de la deuxième face,
   le corps principal comprenant une zone de liaison entourant le premier orifice,
- une capsule, la capsule étant configurée pour se placer dans le premier orifice du patch et être solidaire de celui-ci, la capsule comprenant des microaiguilles configurées pour être insérées dans la peau pour prélever ou analyser un fluide corporel du porteur d'un système de surveillance corporelle lorsque ce dernier est positionné sur un membre d'un utilisateur,

l'ensemble comprenant une couche adhésive recouvrant la zone de liaison sur la deuxième face et liant le corps principal à la capsule de manière non amovible,
le patch (250) présentant une longueur *l* maximale plus élevée d'au moins 2 mm que la longueur d maximale de la capsule (220).

Le système peut avantageusement comprendre les caractéristiques suivantes, prises individuellement ou en l'une quelconque de leurs combinaisons techniquement possibles :
- la première face comprend une texturation configurée pour diminuer l'adhérence entre le patch et des poils de l'utilisateur lorsque les poils adhèrent à l'adhésif recouvrant la première face,
- la texturation comprend un réseau de deuxièmes orifices débouchant sur la première face, le diamètre de chaque deuxième orifice étant supérieur à 0,1 mm, préférablement supérieur à 0,5mm,
- les deuxièmes orifices forment ajours entre la première face et la deuxième face,
- selon l'invention, la couche adhésive recouvrant la zone de liaison est configurée pour lier le corps principal à la capsule de manière non-amovible,
- le patch présente une longueur *l* maximale plus grande que la longueur *d* maximale de la capsule,
- la zone de liaison présente une complémentarité de forme avec la capsule, et préférentiellement forme un bossage du corps principal, la capsule présentant un enfoncement d'une forme complémentaire au bossage,
- le premier orifice comprend, sur une paroi interne, un sillon ou des renfoncements ponctuels, configurés pour recevoir une capsule,
- la zone de liaison présente une dureté en shore plus élevée que la dureté du reste du corps principal,
- l'adhésif est à base de silicone ou à base d'acrylique,
- la zone de liaison est au moins deux fois plus épaisse que le reste du corps principal,
- l'adhésif comprend une pluralité d'ouvertures traversantes, et la taille des deuxièmes orifices du corps principal est supérieure à la taille des ouvertures de l'adhésif, et/ou la densité de surface de deuxièmes orifices du corps principal est supérieure à la densité de surface d'ouverture de l'adhésif, et/ou la densité de deuxièmes orifices du corps principal est supérieure à la densité d'ouvertures de l'adhésif,

Un autre aspect de l'invention est un système de surveillance corporelle, destiné à être attaché à un membre, comprenant :
- un ensemble conforme à l'invention,
- un boitier, apte à être couplé avec la capsule, à l'intérieur duquel se trouve une batterie et un processeur, le processeur étant configuré pour traiter des données obtenues à l'aide du prélèvement ou de la mesure faite par les microaiguilles,
- une lanière, configuré pour maintenir le boitier en place sur le membre, dans lequel la capsule est positionnée au sein du premier orifice du patch.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre schématiquement, en vue éclatée, un bracelet, un boitier, une capsule et un patch tel qu'utilisable dans le cadre de l'invention,
- la figure 2 illustre une vue en trois dimensions, d'un patch et d'une capsule attachée au patch,
- la figure 3 illustre une vue en coupe de la figure 2.

Sur l'ensemble des figures, les éléments similaires portent des références identiques.

### DESCRIPTION DETAILLEE DE L'INVENTION

### Architecture générale

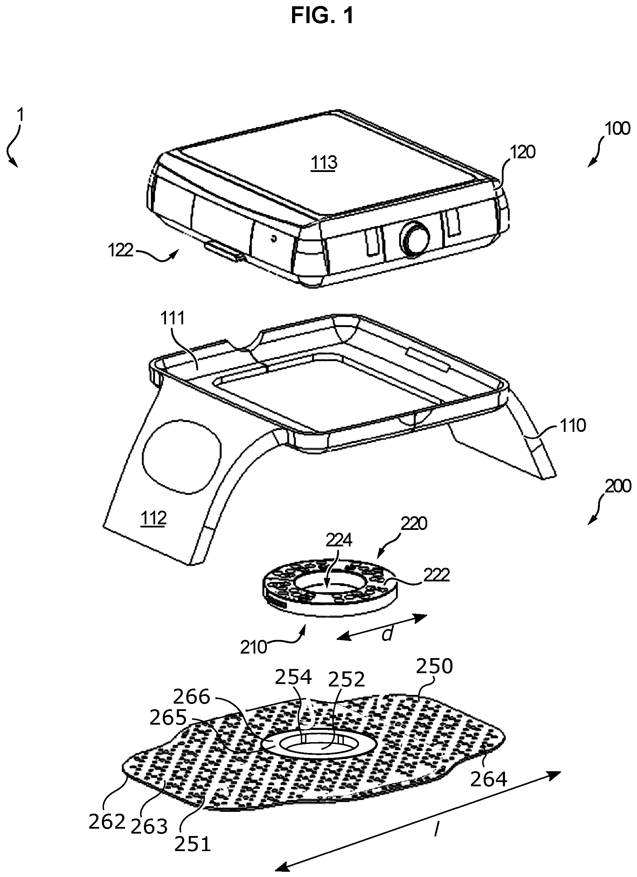

En référence aux figures 1 à 3, la présente invention concerne un système électronique 1 de surveillance corporelle. Il s'agit d'une amélioration du dispositif du document WO2018104647. Par conséquent, l'invention se place dans le même concept général de système intégral autonome à faible douleur, faible risque hygiénique et réutilisable.

Par surveillance corporelle, on entend la vérification de constantes biochimiques d'une personne porteuse du système 1, typiquement la concentration en une protéine, une hormone, un marqueur, en oxygène, en nutriments, dans le liquide interstitiel de la personne. On citera l'exemple de la glycémie. L'homme du métier pourra surveiller si besoin d'autres grandeurs physiques corporelles telles que le lactate, l'hydratation, *etc.*

La description sera illustrée avec du liquide interstitiel mais s'applique aux autres liquides corporels tels que le sang.

Le système 1 est dit autonome, car il ne nécessite pas l'utilisation de matériel supplémentaire.

Le système 1 est destiné à être attaché à un membre d'un être vivant, typiquement un bras ou une jambe d'un être humain. La zone d'attache privilégiée est le poignet où le système 1 s'apparente à une montre.

Le système 1 est formé de deux modules 100, 200 (figure 1) reliées entre eux par une liaison séparable 300 qui est réutilisable. On définit ainsi une position couplée et une position libre. En position couplée, les deux modules 100, 200 ne sont pas séparés physiquement et peuvent échanger des données.

Le premier module 100 comprend notamment des moyens d'attache et de serrage 110 du système 1 à un membre (qu'on appellera lanière 112 ou bracelet) et le deuxième module 200 comprend une capsule 220 qui intègrent des microaiguilles 210 configurées pour être insérées dans la peau (dans une partie superficielle de l'épiderme). Ces microaiguilles 210, lorsque le premier module 100 est en position sur le membre, permettent de prélever et/ou d'analyser un fluide corporel, comme mentionné précédemment.

Les microaiguilles consistent avantageusement en un réseau de microaiguilles 210 au contact de la peau lorsque la capsule 220 est placée sur le corps d'une personne. Les microaiguilles 210 peuvent donc être soit creuses, pour prélever du liquide, soit pleines, pour analyser directement le liquide. Dans le premier cas, typiquement, les microaiguilles 210 permettent l'extraction de liquide interstitiel du derme de façon indolore sans perlement de sang, et l'envoie vers un capteur dans le système 1 (plus précisément préférablement dans le deuxième module 200). Dans le deuxième cas, les microaiguilles 210 ne prélèvent pas de fluide et intègrent le capteur sur leur surface, sous la forme d'un matériau biochimique apte à réagir avec l'analyte que l'on souhaite mesurer dans le fluide. Dans le cas de microaiguilles percées formant un canal dans chaque microaiguille, un prélèvement peut être réalisé en connectant de manière fluidique un système de pompage du fluide interstitiel au canal, ou simplement par capillarité. Un système d'analyse peut comprendre des microaiguilles chacune pourvue d'une électrode ou d'un ensemble d'électrodes, ou être déporté après les microaiguilles, de sorte à entraîner une réaction électrochimique adaptée à détecter un analyte dans le fluide interstitiel.

De façon préférée, la capsule 220 comprend entre quatre et cinquante microaiguilles, sensiblement pyramidales, avec des pointes d'une hauteur comprise entre 100µm et 1000µm, préférablement 0.3 mm et 0.8 mm. Chacune de ces caractéristiques avantageuses des microaiguilles 210 peut être prise séparément ou en combinaison avec les autres.

Les deux modules 100 et 200 présentent chacun une face de couplage 122, 222, de forme complémentaire, qui permet de placer le deuxième module 200 dans un emplacement d'accueil du premier module 100

Enfin, le premier module 100 et le deuxième module 200 sont configurés pour être couplés par une liaison séparable 300.

Comme illustré sur la figures 1, le premier module 100 comprend en outre un boitier 120 dans lequel sont disposés des moyens de traitement de données (en particulier un processeur ou un microcontrôleur) configurés pour traiter des mesures acquises par le capteur, et le cas échéant des moyens de stockage de données (notamment une mémoire, en particulier de type flash, et/ou la mémoire du microcontrôleur) permettant par exemple de stocker ces mesures, et/ou une date de la première utilisation de chaque capteur pour calculer une date de péremption du ou des capteurs (les capteurs biochimiques ont une durée de vie limitée). Les moyens de traitement de données servent aussi à générer des consignes vers différentes composants. Dans le cadre de cette description, ces différentes fonctions sont assurées par une même unité. Toutefois, il est possible de prévoir des processeurs dédiés. Le système comprend également une batterie pour l'alimentation électrique des composants, avantageusement rechargeable, par exemple via un port (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran 113, éventuellement tactile pour afficher les résultats de la surveillance à l'utilisateur.

L'homme du métier connait des algorithmes de traitement de mesures de capteurs 24 et des interfaces associées, et saura les implémenter dans le présent système 1.

Le boitier 120 comprend en outre des connecteurs électriques, sur sa face de couplage 122, qui se couple avec la face de couplage 222 de la capsule 220.

La capsule 220 du deuxième module 200 a une forme de boite fermée, typiquement étanche, qui peut se coupler avec le boitier 120. Cette capsule 220 est interchangeable, ce qui permet d'obtenir un système économique et efficace, où seules les parties dites consommables doivent être changées. La capsule 220 peut avoir une forme annulaire, avec une ouverture traversante 224 au centre. Dans une variante mentionnée précédemment, le capteur est positionné à l'intérieur de la capsule 220 (ou alors dans le boitier 120) et analyse le fluide prélevé par les microaiguilles. La capsule 220 comprend des connecteurs électriques, sur une face de couplage 222 avec le boitier 120, qui peuvent coopèrent avec les connecteurs électriques du boitier 120. S'il y a transmission de fluide depuis le deuxième module vers le premier alors des connecteurs fluidiques complémentaires sont prévus sur la capsule 220 et le boitier 120.

Enfin, le deuxième module 200 comprend un patch 250, solidaire de façon amovible à la capsule 220, qui est décrit en détail ci-dessous.

Le deuxième module 200 (capsule 220 et patch 250) forme un ensemble interchangeable du système qui est choisi selon le type de surveillance voulu et en fonction de l'état de détérioration des microaiguilles 210 et/ou du capteur.

En effet, dans la mesure où la capsule 220 contient les microaiguilles 210 et/ou le capteur (notamment dans le cas de microaiguilles qui prélèvent), changer de capsule 220 permet de changer de matériel si celui-ci est en fin de vie ou si l'on souhaite changer de grandeur physique mesurée, en une manipulation simple, rapide et sure, sans devoir jeter d'autres parties (en particulier le premier module 100).

Et dans la mesure où la capsule 220 minimise la quantité d'élément et/ou matériaux coûteux (équipement électronique avancé tel qu'une batterie ou des moyens de communication sans fil), elle est relativement peu chère.

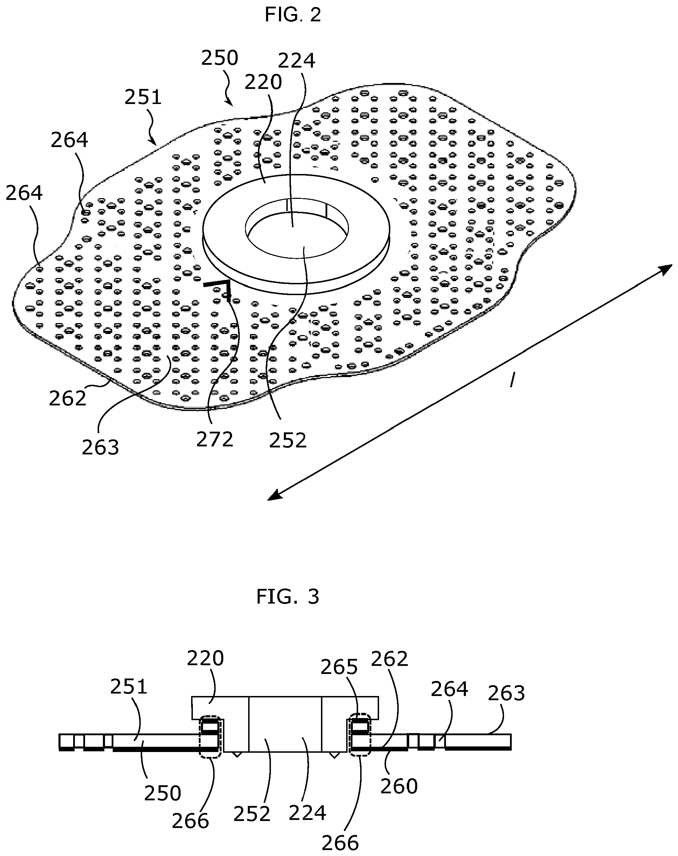

### Patch 250 (figures 2 et 3)

Le patch 250 fonctionne comme un adhésif pour maintenir les microaiguilles 210 enfoncées dans la peau. La capsule 220 étant attachée au patch 250, elle profite du pouvoir adhésif du patch 250 pour être maintenue contre la peau.

Le patch 250, peu coûteux, peut être changé plus fréquemment que la capsule 220. Cela est rendu possible grâce à la fixation amovible entre le patch 250 et la capsule 220.

Il est conçu pour ressembler à un pansement, afin que les personnes qui le portent ne se sentent pas stigmatisés par le port d'un élément inhabituel.

Le patch 250 a une longueur *l* comprise entre 3cm et 10cm, une largeur comprise entre 3cm et 10cm et une épaisseur e comprise entre 0,1cm et 1cm.

Le patch 250 comprend un corps principal 251 avec un orifice, dit premier orifice 252, configuré pour recevoir la capsule 220, et avantageusement un adhésif 260 (si le ou les matériaux du patch ne fonctionnent pas déjà comme un adhésif par exemple). Par central, on entend que le premier orifice 252 se trouve intégralement au sein du corps principal 251, et non pas sur un bord. Néanmoins, « central » ne signifie pas nécessairement « centré » : il se peut que le premier orifice 252 ne soit pas centré sur le corps principal 251.

Le premier orifice 252 fait au moins 2mm de diamètre (ce n'est donc pas un micro-orifice qui permet d'aérer le patch).

Le corps principal 251 est souple, c'est-à-dire que l'application d'un moment sur le corps principal 251 par l'utilisateur entraîne une torsion du corps principal 251, et non une cassure.

En effet, à l'instar d'un pansement, la souplesse du corps principal 251 permet d'épouser le contour du membre et en particulier du poignet, ce qui assure une bonne adhérence.

Le corps principal 251 comprend une première face 262 et un adhésif 260 recouvrant la première face 262. Le corps principal 251 comprend une deuxième face 263 opposée à la première face 262. Le premier orifice 252 forme un ajour au travers de la première face 262 et de la deuxième face 263. Cet ajour est ainsi adapté à recevoir la capsule 220.

Le corps principal 251 comprend une zone de liaison 266 entourant le premier orifice 252. L'ensemble comprend une couche adhésive 265 (illustrée en figure 3) recouvrant la zone de liaison 266 sur la deuxième face 263 et liant le corps principal 251 à la capsule 220. La couche adhésive 265 permet de solidariser, selon l'invention de manière non amovible, le patch 250 à la capsule 220.

De plus, pour éviter l'arrachement involontaire des microaiguilles 210 de la peau, il est important que les efforts d'arrachement qui s'exercent sur le patch 250 ne soit pas transmis à la capsule 220, ou du moins soient atténués. A cet égard, en référence à la figure 3, la zone de liaison 266 présente une complémentarité de forme avec la capsule 220, et préférentiellement forme un bossage du corps principal 251, la capsule 220 présentant un enfoncement d'une forme complémentaire au bossage. Ainsi, les contraintes transmises aux microaiguilles 210 peuvent être atténuées. Le bossage peut être formé par l'ajout d'une bande adhésive au corps principal 251. Préférentiellement, il est possible de former le bossage et la couche adhésive 265 par un ajout d'un adhésif double face à un corps principal comprenant initialement une seule couche.

Vu de profil (**figure 3**), le patch 250 présente une première face 262, préférentiellement plane, configurée pour être au contact de la peau par l'intermédiaire de l'adhésif 260, et une deuxième face 263, opposée à la première face 262 par rapport au corps principal.

La zone de liaison peut être réalisée dans un matériau différent du matériau utilisé pour le reste du corps principal 251, aux caractéristiques physiques différentes. La zone de liaison 266 peut présenter une dureté en shore plus élevée que la dureté du reste du corps principal 251. La dureté shore du corps principale (zone de liaison inclue) peut être préférentiellement comprise entre 20 et 40 shore. Le corps principal peut par exemple comprendre de la silicone.

### Le patch 250 et la capsule 220

Préférentiellement, le patch 250 présente une longueur *l* maximale plus grande que la longueur *d* maximale de la capsule 220. Ainsi, le patch, et particulièrement le corps principal 251 peut épouser la forme du membre et gêne le moins possible l'utilisateur dans sa vie quotidienne. En effet, dans le cas d'un poignet, la capsule 220 est positionnée sur une région plutôt plate du membre, alors que le patch 250 s'étend jusqu'au courbure du poignet.

Pour solidariser la capsule 220 au patch 250 (ou l'inverse) en plus de la fixation par la couche d'adhésif 265, le premier orifice 252 peut comprendre, sur sa paroi interne formée par le corps principal 251, un sillon ou des renfoncement ponctuels 254. Ils fonctionnent comme des encoches pour recevoir une protubérance 228 sur une paroi annulaire extérieur de la capsule 220. Préférablement, l'agencement des encoches 254 et des protubérances 228 est tel qu'il empêche la rotation de la capsule 220 au sein de l'orifice central 252 du patch 250. Une solution préférentielle consiste à prévoir une ou plusieurs encoches ponctuelles, qui bloquent la rotation.

### Le patch 250 et la lanière/bracelet 110

Lorsque les deux modules 100, 200 sont assemblés, le boitier 120 et le patch 250 sont en contact, préférentiellement au niveau la zone de liaison 266 qui est autour de l'ouverture 252 (voir figure 3). Cela permet une première reprise d'effort vers la capsule 220, via le patch 250 quand la zone de liaison présente une dureté plus élevée que celle du reste du corps principal 251.

La reprise d'effort par le bracelet 110 présente deux intérêts : un premier intérêt est que l'effort vient du bracelet 110 lui-même, que l'on serre sur son poignet ou son membre (transmission directe), et un deuxième intérêt vient du fait que le système 1 peut être porté sans boitier 120. Dès lors, pour bien tenir le patch 250, l'effort doit être repris par le bracelet 110 et non le boitier 120.

### Adhésif 260 et couche adhésive 265

L'adhésif 260 est sous forme d'une couche plane, recouvrant la première face 262, du côté destiné à être sur la peau.

L'adhésif 260 utilisé est avantageusement à base de silicone pour sa capacité à pouvoir être décollé et recollé, pour les faibles irritations qu'il provoque, pour la meilleure gestion des douches et l'absence de trace sur la peau. De plus, le silicone fonctionne très bien en arrachement latéral et peu en traction, mais il s'agit du bracelet/lanière qui assure cette fonction. On peut alternativement utiliser un adhésif à base d'acrylique, qui possède un pouvoir adhérant plus fort que le silicone mais qui n'est pas décollable et recollable. Pour protéger l'adhésif du patch 250 (conditionnement, transport, etc.), l'adhésif 260 peut être recouvert d'une couche de papier pelable. L'ensemble comprend une couche adhésive 265 recouvrant la zone de liaison 266 sur la deuxième face 263 et liant le corps principal 251 à la capsule 220.

La couche adhésive 265 est configurée pour lier le corps principal 251 à la capsule 220 de manière non-amovible. Ainsi, l'ensemble formé par le patch 250 et par la capsule 220 est à usage unique, ce qui assure une simplicité d'utilisation pour l'utilisateur.

Une telle liaison non-amovible peut être mise en œuvre par différents moyens. La couche adhésive 265 peut être formée par une colle liquide ou par une colle solide. La couche adhésive 265 peut être formée par une colle acrylique. La couche adhésive 265 peut être formée par un traitement aux ultrasons d'une surface de la zone de liaison 266 et de la capsule 220 destinée à être en contact.

### Respirabilité & poils

Pour faciliter la respiration de la peau afin d'éviter les effets de macération et limiter l'arrachage de poils, le corps principal 251 peut comprendre une texturation configurée pour diminuer l'adhérence entre le patch 250 et des poils de l'utilisateur lorsque les poils adhèrent à l'adhésif 260 recouvrant la première face 262. En effet, l'adhésif 260 recouvre la première face 262, ce qui entraîne une texturation de l'adhésif 260 lui-même. Ainsi, lorsqu'un poil est mis en contact avec l'adhésif 260, la texturation entraîne une pluralité de points de contact locaux entre l'adhésifs 260 et le poil, plutôt qu'un contact continu entre l'adhésif 260 et le poil, permettant de diminuer l'adhérence entre le poil et le patch.

De préférence, la dimension minimale d'un motif de texturation est petite devant la taille d'un poil, préférentiellement la dimension minimale d'un motif de texturation est inférieure à 0,5 mm.

La texturation de la première face peut être mise en œuvre par plusieurs moyens. La texturation peut comprendre des stries s'étendant le long de la première face 262. La texturation peut également comprendre une pluralité d'enfoncements ponctuels dans la première face 262. La texturation peut comprendre un réseau de deuxièmes orifices 264 débouchant sur la première face 262, le diamètre de chaque deuxième orifice 264 étant supérieur à 0,1 mm, préférablement supérieur à 0,5mm. Préférentiellement, les deuxièmes orifices 264 forment ajours entre la première face 262 et la deuxième face 263.

L'adhésif 260 peut comprendre des ouvertures traversantes (visibles en figure 3), pour que la communication d'air se fasse depuis la peau et limite fortement la surface de colle sur les poils réduisant le nombre de poils arrachés au moment du retrait du patch adhésif.

La surface majeure de contact d'adhésif avec la peau se retrouve majoritairement à l'extérieur de la capsule (220), permettant ainsi de réaliser les deuxièmes orifices.

Le patch adhésivé doit être très collant pour résister à une semaine de douche/bain ou plus. On perce de part en part le patch afin de diminuer la surface de collage sur les poils, et donc la force d arrachement des poils.

En outre, l'ensemble des trous redonne ainsi de la souplesse au patch qui a ainsi plus de conformabilité à la peau et aux différentes formes de poignet.

Les zone de patch latérales sont en compression forte du fait des parois de la montre ce qui colle fortement les poils et augmentent la proportion de poils arrachés dans cette zone. Pour résoudre cela, le patch 250 présente des bordures de part et d'autre du premier orifice 252 en regard du boitier, les bordures du patch 250 ayant la forme de dents de scie. La hauteur des incrémentations des dents de scie est de 50% voire plus de la largeur de l'appui correspondant.

Pour s'assurer que les ouvertures de l'adhésif 260 communiquent avec les deuxièmes orifices 264 du corps principal 251, la taille des deuxièmes orifices 264 du corps principal 251 est supérieure à la taille des ouvertures de l'adhésif 260, et/ou la densité de surface de deuxièmes orifices 264 du corps principal 251 est supérieure à la densité de surface d'ouverture de l'adhésif 260, et/ou la densité de deuxièmes orifices 264 du corps principal est supérieure à la densité d'ouvertures de l'adhésif 260. Ainsi, toute superposition de l'adhésif sur le corps principal génère une communication entre les ouvertures, sans qu'il ne soit nécessaire de chercher à aligner des ouvertures entre elles.

Enfin, pour des questions d'esthétisme (absence de visibilité de la peau quand le patch est porté seul), les ouvertures du corps principal peuvent être inclinées (par rapport à un plan formé par le patch 250 quand il est mis à plat).

Enfin, le patch 250 comprend un signe distinctif 272 aux abords de l'ouverture afin de pouvoir orienter aisément la capsule 220 dans le patch 250. Ce signe distinctif est représenté par une flèche 272 sur les figures. Deux flèches 272 peuvent être prévues, une de chaque côté, en fonction du sens de mise en place de la capsule 220. Un signe similaire peut être présent sur la capsule 220. Les signes entre le patch 250 et la capsule 220 sont préférablement identiques ou symétriques.

Les signes distinctifs 272 peuvent se retrouver sous le patch 250, côté peau. De la même façon, les signes entre le patch 250 et la capsule 220 sont préférablement identiques ou symétriques.

Le sens de la flèche peut changer entre les deux faces du patch pour que visuellement l'utilisateur mettre la capsule dans le bon sens (ie. mettre les microaiguilles côté peau du patch).

### Fixation

Pour pouvoir attacher le patch 250 au boitier 120, des aimants ou matériaux ferromagnétiques 304 peuvent être situés dans le corps principal 251, préférentiellement dans la zone de liaison, de préférence de façon régulière autour de l'ouverture 252. Un adhésif supplémentaire peut également être prévu pour attacher le patch 250 au boitier 120.

### Kit de patchs

Du fait de sa fonction, le patch 250 est amené à être changé plus souvent que la capsule. Par conséquent, il peut être fourni en plusieurs exemplaires, tous identiques. Alternativement, comme le patch 250 doit être adapté aux circonstances (type de peau, taille du membre, conditions d'utilisation), la largeur et/ou la longueur et/ou les propriétés de l'adhésif peuvent être différentes entre deux patchs du kit.

De plus, différentes capsules peuvent être utilisées, avec des formes différentes : les patchs peuvent donc avoir des formes de l'orifice 252 différentes.

## Revendications

1. Ensemble pour un système de surveillance corporelle (1), comprenant :
- un patch (250) pour système de surveillance corporelle (1), le patch comprenant un corps principal (251) avec un premier orifice (252) d'au moins 2 mm de diamètre,
le corps principal (251) comprenant une première face (262) et un adhésif (260) recouvrant la première face (262),
le corps principal (251) comprenant une deuxième face (263) opposée à la première face (262), le premier orifice (252) formant un ajour au travers de la première face (262) et de la deuxième face (263),
le corps principal (251) comprenant une zone de liaison (266) entourant le premier orifice (252),
- une capsule (220), la capsule (220) étant configurée pour se placer dans le premier orifice (252) du patch (250) et être solidaire de celui-ci, la capsule (220) comprenant des microaiguilles (210) configurées pour être insérées dans la peau pour prélever ou analyser un fluide corporel du porteur d'un système de surveillance corporelle (1) lorsque ce dernier est positionné sur un membre d'un utilisateur,
l'ensemble comprenant une couche adhésive (265) recouvrant la zone de liaison (266) sur la deuxième face (263) et liant le corps principal (251) à la capsule (220) de manière non amovible,
le patch (250) présentant une longueur *l* maximale plus élevée d'au moins 2 mm que la longueur *d* maximale de la capsule (220).

2. Ensemble selon la revendication 1, dans lequel la première face (262) comprend une texturation configurée pour diminuer l'adhérence entre le patch (250) et des poils de l'utilisateur lorsque les poils adhèrent à l'adhésif (260) recouvrant la première face (262).

3. Ensemble selon la revendication 2, dans lequel la texturation comprend un réseau de deuxièmes orifices (264) débouchant sur la première face (262), le diamètre de chaque deuxième orifice (264) étant supérieur à 0,1 mm.

4. Ensemble selon la revendication 3, dans lequel le diamètre de chaque deuxième orifice (264) est supérieur à 0,5mm.

5. Ensemble selon l'une des revendications 3 à 4, dans lequel les deuxièmes orifices (264) forment ajours entre la première face (262) et la deuxième face (263).

6. Ensemble selon l'une des revendications 1 à 5, dans lequel la zone de liaison (266) présente une complémentarité de forme avec la capsule (220).

7. Ensemble selon l'une des revendications 1 à 5, dans lequel la zone de liaison (266) forme un bossage du corps principal (251), la capsule (220) présentant un enfoncement d'une forme complémentaire au bossage.

8. Ensemble selon l'une des revendications 1 à 7, dans lequel le premier orifice (252) comprend, sur une paroi interne, un sillon ou des renfoncements ponctuels (254), configurés pour recevoir une capsule (220).

9. Ensemble selon l'une des revendications 1 à 8, dans lequel la zone de liaison (266) présente une dureté en shore plus élevée que la dureté du reste du corps principal (251).

10. Ensemble selon l'une des revendications 1 à 9, dans lequel l'adhésif (260) est à base d'acrylique.

11. Ensemble selon l'une quelconque des revendications 1 à 10, dans lequel la zone de liaison (266) est au moins deux fois plus épaisse que le reste du corps principal (251).

12. Ensemble selon l'une quelconque des revendications 1 à 11, dans lequel le patch (250) présente des bordures de part et d'autre du premier orifice (252), les bordures du patch (250) ayant la forme de dents de scie.

13. Ensemble selon l'une quelconque des revendications 3 à 9, dans lequel :
- l'adhésif (260) comprend une pluralité d'ouvertures traversantes,
- la taille des deuxièmes orifices (264) du corps principal (251) est supérieure à la taille des ouvertures de l'adhésif (260), et/ou la densité de surface de deuxièmes orifices (264) du corps principal (251) est supérieure à la densité de surface d'ouverture de l'adhésif (260), et/ou la densité de deuxièmes orifices (264) du corps principal est supérieure à la densité d'ouvertures de l'adhésif (260).

14. Système de surveillance corporelle (1), destiné à être attaché à un membre, comprenant :
- un ensemble selon l'une des revendications précédentes,
- un boitier, apte à être couplé avec la capsule (220), à l'intérieur duquel se trouve une batterie et un processeur, le processeur étant configuré pour traiter des données obtenues à l'aide du prélèvement ou de la mesure faite par les microaiguilles (210)
- une lanière, configuré pour maintenir le boitier (120) en place sur le membre, dans lequel la capsule (220) est positionnée au sein du premier orifice (252) du patch (250).

## Patentansprüche

1. Anordnung für ein Körperüberwachungssystem (1), umfassend:
- ein Pflaster (250) für ein Körperüberwachungssystem (1), wobei das Pflaster einen Hauptkörper (251) mit einer ersten Öffnung (252) von mindestens 2 mm Durchmesser umfasst,
wobei der Hauptkörper (251) eine erste Seite (262) und einen Haftstoff (260) umfasst, der die erste Seite (262) bedeckt,
wobei der Hauptkörper (251) eine der ersten Seite (262) gegenüberliegende zweite Seite (263) umfasst, wobei die erste Öffnung (252) einen Durchbruch durch die erste Seite (262) und die zweite Seite (263) bildet,
wobei der Hauptkörper (251) einen Verbindungsbereich (266) umfasst, der die erste Öffnung (252) umgibt,
- eine Kapsel (220), wobei die Kapsel (220) ausgelegt ist, um in die erste Öffnung (252) des Pflasters (250) eingesetzt zu werden und mit dieser fest verbunden zu sein, wobei die Kapsel (220) Mikronadeln (210) umfasst, die ausgelegt sind, um in die Haut eingeführt zu sein, um ein Körperfluid des Trägers eines Körperüberwachungssystems (1) zu entnehmen oder zu analysieren, wenn dieses an einer Gliedmaße eines Benutzers positioniert ist,
wobei die Anordnung eine Haftschicht (265) umfasst, die den Verbindungsbereich (266) auf der zweiten Seite (263) bedeckt und den Hauptkörper (251) mit der Kapsel (220) nicht lösbar verbindet,
wobei das Pflaster (250) eine maximale Länge *l* aufweist, die mindestens 2 mm größer ist als die maximale Länge *d* der Kapsel (220).

2. Anordnung nach Anspruch 1, wobei die erste Seite (262) eine Texturierung umfasst, die ausgelegt ist, um die Haftung zwischen dem Pflaster (250) und den Haaren des Benutzers zu verringern, wenn die Haare an dem Haftstoff (260) haften, der die erste Seite (262) bedeckt.

3. Anordnung nach Anspruch 2, wobei die Texturierung ein Netzwerk zweiter Öffnungen (264) umfasst, die auf der ersten Seite (262) ausmünden, wobei der Durchmesser jeder zweiten Öffnung (264) größer als 0,1 mm ist.

4. Anordnung nach Anspruch 3, wobei der Durchmesser jeder zweiten Öffnung (264) größer als 0,5 mm ist.

5. Anordnung nach einem der Ansprüche 3 bis 4, wobei die zweiten Öffnungen (264) zwischen der ersten Seite (262) und der zweiten Seite (263) Durchbrüche bilden.

6. Anordnung nach einem der Ansprüche 1 bis 5, wobei der Verbindungsbereich (266) Formkomplementarität mit der Kapsel (220) aufweist.

7. Anordnung nach einem der Ansprüche 1 bis 5, wobei der Verbindungsbereich (266) eine Erhebung des Hauptkörpers (251) bildet, wobei die Kapsel (220) eine Vertiefung mit einer zur Erhebung komplementären Form aufweist.

8. Anordnung nach einem der Ansprüche 1 bis 7, wobei die erste Öffnung (252) an einer Innenwand eine Rille oder punktuelle Vertiefungen (254) aufweist, die zur Aufnahme einer Kapsel (220) ausgelegt sind.

9. Anordnung nach einem der Ansprüche 1 bis 8, wobei der Verbindungsbereich (266) eine höhere Shore-Härte als die Härte des Rests des Hauptkörpers (251) aufweist.

10. Anordnung nach einem der Ansprüche 1 bis 9, wobei der Haftstoff (260) auf Acrylbasis ist.

11. Anordnung nach einem der Ansprüche 1 bis 10, wobei der Verbindungsbereich (266) mindestens doppelt so dick ist wie der Rest des Hauptkörpers (251).

12. Anordnung nach einem der Ansprüche 1 bis 11, wobei das Pflaster (250) Ränder auf beiden Seiten der ersten Öffnung (252) aufweist, wobei die Ränder des Pflasters (250) die Form von Sägezähnen haben.

13. Anordnung nach einem der Ansprüche 3 bis 9, wobei:
- der Haftstoff (260) eine Vielzahl von Durchgangsöffnungen umfasst,
- die Größe der zweiten Öffnungen (264) des Hauptkörpers (251) größer ist als die Größe der Öffnungen des Haftstoffs (260), und/oder die Oberflächendichte der zweiten Öffnungen (264) des Hauptkörpers (251) größer ist als die Öffnungsoberflächendichte des Haftstoffs (260), und/oder die Dichte der zweiten Öffnungen (264) des Hauptkörpers größer ist als die Öffnungsdichte des Haftstoffs (260).

14. Körperüberwachungssystem (1), das zur Anbringung an einer Gliedmaße bestimmt ist, umfassend:
- eine Anordnung nach einem der vorstehenden Ansprüche,
- ein Gehäuse, das mit der Kapsel (220) gekoppelt werden kann, in dessen Inneren sich eine Batterie und ein Prozessor befinden, wobei der Prozessor ausgelegt ist, um Daten zu verarbeiten, die anhand der Entnahme oder Messung durch die Mikronadeln (210) gewonnen wurden,
- einen Riemen, der ausgelegt ist, um das Gehäuse (120) an der Stelle an der Gliedmaße zu halten, in dem die Kapsel (220) in der ersten Öffnung (252) des Pflasters (250) positioniert ist.

## Claims

1. An assembly for a body monitoring system (1), comprising:
- a patch (250) for a body monitoring system (1), the patch comprising a main body (251) with a first opening (252) of at least 2 mm in diameter,
the main body (251) comprising a first face (262) and an adhesive (260) covering the first face (262),
the main body (251) comprising a second face (263) opposite the first face (262), the first opening (252) forming an aperture through the first face (262) and the second face (263),
the main body (251) comprising a bonding area (266) surrounding the first opening (252),
- a capsule (220), the capsule (220) being configured to be placed in the first opening (252) of the patch (250) and to be integral therewith, the capsule (220) comprising microneedles (210) configured to be inserted into the skin to collect or analyse a bodily fluid from the wearer of a body monitoring system (1) when the latter is positioned on a limb of a user,
the assembly comprising an adhesive layer (265) covering the bonding area (266) on the second face (263) and bonding the main body (251) to the capsule (220) in a non-removable manner,
the patch (250) having a maximum length *l* that is at least 2 mm greater than the maximum length *d* of the capsule (220).

2. Assembly according to claim 1, wherein the first face (262) comprises a texture configured to decrease adhesion between the patch (250) and the user's hair when the hairs adhere to the adhesive (260) covering the first face (262).

3. Assembly according to claim 2, wherein the texturing comprises an array of second apertures (264) opening onto the first face (262), the diameter of each second aperture (264) being greater than 0.1 mm.

4. Assembly according to claim 3, wherein the diameter of each second orifice (264) is greater than 0.5 mm.

5. Assembly according to one of claims 3 to 4, wherein the second orifices (264) form openings between the first face (262) and the second face (263).

6. Assembly according to one of claims 1 to 5, wherein the connection area (266) is complementary in shape to the capsule (220).

7. Assembly according to one of claims 1 to 5, wherein the connection area (266) forms a boss on the main body (251), the capsule (220) having a recess of a shape complementary to the boss.

8. Assembly according to one of claims 1 to 7, wherein the first orifice (252) comprises, on an inner wall, a groove or punctual recesses (254), configured to receive a capsule (220).

9. Assembly according to one of claims 1 to 8, wherein the bonding area (266) has a higher Shore hardness than the hardness of the rest of the main body (251).

10. Assembly according to one of claims 1 to 9, wherein the adhesive (260) is acrylic-based.

11. Assembly according to any one of claims 1 to 10, wherein the bonding area (266) is at least twice as thick as the rest of the main body (251).

12. An assembly according to any one of claims 1 to 11, wherein the patch (250) has edges on either side of the first opening (252), the edges of the patch (250) being sawtooth-shaped.

13. An assembly according to any one of claims 3 to 9, wherein:
- the adhesive (260) comprises a plurality of through-openings,
- the size of the second apertures (264) of the main body (251) is greater than the size of the apertures of the adhesive (260), and/or the surface density of second orifices (264) of the main body (251) is greater than the surface density of openings of the adhesive (260), and/or the density of second orifices (264) of the main body is greater than the density of openings of the adhesive (260).

14. A body monitoring system (1), for attachment to a limb, comprising:
- an assembly according to one of the preceding claims,
- a housing, capable of being coupled with the capsule (220), inside which there is a battery and a processor, the processor being configured to process data obtained using the sampling or measurement made by the microneedles (210)
- a strap, configured to hold the housing (120) in place on the limb,
wherein the capsule (220) is positioned within the first opening (252) of the patch (250).
